(19)

**Europäisches Patentamt**
**European Patent Office**
**Office européen des brevets**

(11) **EP 4 640 246 A1**

(12) **EUROPEAN PATENT APPLICATION**

(43) Date of publication:
**29.10.2025 Bulletin 2025/44**

(21) Application number: **24382462.0**

(22) Date of filing: **25.04.2024**

(51) International Patent Classification (IPC):
***A61L 15/22*** *(2006.01)*      ***A61L 15/44*** *(2006.01)*
***A61L 15/46*** *(2006.01)*

(52) Cooperative Patent Classification (CPC):
(C-Sets available)
**A61L 15/225; A61L 15/44; A61L 15/46;**
A61L 2300/104; A61L 2300/404        (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Viscofan España, S.L.U.**
**31192 Tajonar (ES)**

(72) Inventors:
• **IZCO ZARATIEGUI, Jesús Maria**
**31192 MUTILVA (ES)**
• **LIZCANO DE VEGA, Lorenzo**
**31110 NOÁIN (ES)**

(74) Representative: **ZBM Patents - Zea, Barlocci &
Markvardsen**
**Rambla de Catalunya, 123**
**08008 Barcelona (ES)**

(54)   **A TOPICAL DRESSING COMPRISING A POLYAMIDE/PVP FILM**

(57)   It relates to a topical dressing comprising: a) a film; b) a pressure-sensitive biocompatible adhesive on one of the surfaces of the film; and c) a release liner with non-stick properties adhered to the adhesive surface of the film; wherein the film comprises one or more layers and each layer comprises a polyamide in an amount from 52-92 % by weight; and polyvinyl pyrrolidone in in an amount from 8-30 % by weight; and the sum of components of each of the layers being 100% by weight, which is suitable for wound healing. It also relates to its preparation process.

EP 4 640 246 A1

(52) Cooperative Patent Classification (CPC): (Cont.)

    C-Sets
    **A61L 15/225, C08L 39/06;**
    **A61L 15/225, C08L 77/00**

## Description

### Technical Field

The invention relates to the field of polymeric films and dressings made of these films. It also relates to their preparation process and their uses as dressings.

### Background Art

Topical dressings play a crucial role in different fields such as in the medical field by providing protective barriers or therapeutic agents to wounds, burns, or other skin conditions. The material's choice and its composition critically influence the dressing's efficacy by determining its water vapor permeability, flexibility, adhesiveness, and overall therapeutic benefits.

Patent document WO2011022680A2 discloses wound dressings that include a polymer matrix integrated with nitric oxide-releasing polysiloxane macromolecules. These dressings, whether within or on the polymer matrix, have shown therapeutic efficacy. However, while this document offers a significant advancement in the field, certain aspects, such as optimizing oxygen transmission rate, elasticity, and tensile strength, can still be enhanced.

Blends of polyamide with polyvinylpyrrolidone have been used for preparing polymeric films in a very different field, specifically, in the field of food products, having properties appropriate for such field. For instance, EP1380212B1 describes a heat-shrinkable sausage casing film, which is made of a blend of polyamide and cross-linked polymer of N-vinylpyrrolidone, with moderate water vapor permeability and moderate oxygen impermeability but allows the passage of smoke products and thus allows the sausage to be smoked.

On the other hand, mixtures of polyamides with non-cross-linked polyvinyl pyrrolidone have been also reported in WO20231803231A1 for preparing thermoplastic films and packaging for sterilization of objects or materials, in particular, with water vapor or ethylene oxide.

Despite the advancements in the field of topical dressings, there is still a pressing need for further research, innovation, and development in this domain. The current state of the art has yet to offer a product that seamlessly integrates the strengths of both materials, addressing their individual limitations while synergistically enhancing their beneficial properties. This points towards, laying the groundwork for the present invention's objectives and contributions.

### Summary of Invention

The inventors have developed a topical dressing comprising a film primarily made of polyamide and polyvinylpyrrolidone (PVP). The integration of these polyamide and polyvinylpyrrolidone in topical dressing has led to dressings with enhanced characteristics. The film, designed as a topical dressing, boasts flexibility, has high water vapor permeability, and minimal oxygen permeability. Moreover, the data of in vivo tests show good water vapor permeability in the absence of skin reactions. These characteristics render it ideally suited for the purposes of the present invention.

Polyamide and polyvinylpyrrolidone mixtures have found applications in various sectors, such as the food industry. Yet, to the knowledge of the inventors, the unique composition of this film, whether with or without plasticizers, has not been previously disclosed for the intent of the current application. Furthermore, the film disclosed herein provides excellent properties for topical dressings which outperform inventions disclosed in the art.

Thus, a first aspect of the present invention relates to a topical dressing comprising a film, wherein the film comprises one or more layers and each layer comprises a polyamide in an amount from 52-92 % by weight; and polyvinyl pyrrolidone in in an amount from 8-30 % by weight; and the sum of components in each layer being 100% by weight.

A second aspect of the present invention relates to the use of a topical dressing as defined above for protecting a wound.

A third aspect of the present invention relates to a topical dressing as defined above, further comprising an antimicrobial agent in the surface of the layer to be in contact with the skin, for use in the prevention and/or treatment of a microbial infection.

A fourth aspect of the present invention relates to the use of the topical dressing as defined above for protecting the skin.

A fifth aspect of the present invention relates to a film which comprises one or more layers, and each layer comprises a polyamide in an amount from 60-92 % by weight; polyvinyl pyrrolidone in in an amount from 8-30 % by weight, and an elastomer in an amount form 15-40% by weight; and the sum of components in each layer being 100% by weight.

### Brief Description of Drawings

FIG. 1 shows a comparative graph of TEWL averages on the skin of example 3b and film C (Commercial film) against the times t0 (before placing the films) and t2final (after removing the films).

FIG. 2 shows a comparative graph of TEWL averages obtained on example 3b and film C (commercial film) against the initial or flash times (immediately after placing the films), after 1 hour and after 2 hours.

FIG. 3 shows the differences between the means of the TEWL measures of the 3b and C films.

## Detailed description of the invention

All terms as used herein in this application, unless otherwise stated, shall be understood in their ordinary meaning as known in the art. Other more specific definitions for certain terms as used in the present application are as set forth below and are intended to apply throughout the description and claims.

As used herein, the term "optional" means that the element or step to which this term refers, may or may not be present.

Where in the present invention a numerical interval is used, this includes the values of the extremes of the interval. In particular, as used herein, the term "comprised between" refers to a range of values including the end points of the range.

Numeric examples given in the form "x to y" include the values given. When multiple preferred numeric ranges are specified in this format, all ranges created by combining the various endpoints are also included.

The word "comprise" for the purposes of the present invention encompasses the case of "consisting of".

Unless otherwise stated, all percentages mentioned herein are expressed in weight with respect to the total weight of the product, provided that the sum of the amounts of the components is equal to 100%.

The terms "at least one" or "one or more" as used herein refers to 1 or more, for example 2, 3, 4, 5, 6, 7, 8, 9 or more.

As used herein, the term "monolithic" film may comprise any film that is continuous and substantially free or free of pores. In certain alternative embodiments of the invention, a "monolithic" film may comprise fewer pore structures than would otherwise be found in a microporous film. Monolithic films are continuous and free of pores.

The term "permeable" refers to a film with a water vapor transmission rate of at least 170 g/m$^2$ per day measured for the film before sterilization by the method detailed in the examples section.

The term Trans-epidermal Water Loss (TEWL) refers to the measurement of water vapor loss through the epidermis to the surrounding atmosphere. It is an indicator of the skin's barrier function and is a relevant parameter for assessing the performance of dressings in maintaining optimal moisture conditions.

As mentioned above, it is part of the present invention a topical dressing comprising a film, wherein the film comprises one or more layers and each layer comprises a polyamide in an amount from 52-92 % by weight; and polyvinyl pyrrolidone in in an amount from 8-30 % by weight; and the sum of components in each layer being 100% by weight. This topical dressing is suitable for the treatment of a wound.

In a particular embodiment, the topical dressing is that which comprises a film, wherein the film comprises one or more layers and each layer comprises a polyamide in an amount from 60-92 % by weight; and polyvinyl pyrrolidone in in an amount from 8-30 % by weight; and the sum of components in each layer being 100% by weight.

In another particular embodiment the topical dressing is that which comprises a film, wherein the film comprises one or more layers and each layer consists of a polyamide in an amount from 70-92 % by weight; and polyvinyl pyrrolidone in in an amount from 8-30 % by weight; and the sum of components in each layer being 100% by weight.

The topical dressing is a material applied directly to a wound or damaged skin to promote healing, protect from infection, and maintain a moist environment that facilitates the natural healing process. These dressings can vary widely in type and function, depending on the nature and severity of the wound. In a particular embodiment, the topical dressing is selected from the group consisting of bandage, pad, compress, medical tape, sticking plaster, and adhesive bandage.

In a particular embodiment the topical dressing is that which comprises: a) a film; b) a pressure-sensitive biocompatible adhesive on one of the surfaces of the film; and c) a release liner with non-stick properties adhered to the adhesive surface of the film; wherein: the film comprises one or more layers and each layer comprises a polyamide in an amount from 52-92 % by weight; and polyvinyl pyrrolidone in in an amount from 8-30 % by weight; and the sum of components of each of the layers being 100% by weight.

In another particular embodiment the topical dressing is that which comprises: a) a film; b) a pressure-sensitive biocompatible adhesive on one of the surfaces of the film; and c) a release liner with non-stick properties adhered to the adhesive surface of the film; wherein: the film comprises one or more layers and each layer comprises a polyamide in an amount from 60-92 % by weight; and polyvinyl pyrrolidone in in an amount from 8-30 % by weight; and the sum of components of each of the layers being 100% by weight.

A biocompatible adhesive is an adhesive having the ability to be in contact with a living system without producing an adverse effect.

EP 4 640 246 A1

In a particular embodiment, the topical dressing according to the present invention is that where the film comprises multiple layers and the adhesive is on the surface of the layer to be in contact with the skin.

In a particular embodiment, the topical dressing according to the present invention is that where the polyamide is in an amount from 65 to 92% by weight. In a particular embodiment, the topical dressing according to the present invention is that where the polyamide is in an amount from 70 to 90% by weight. In another particular embodiment, the topical dressing according to the present invention is that where the polyamide is in an amount in the range of from 70 to 85% by weight. In another particular embodiment, the topical dressing according to the present invention is that where the polyamide is in an amount in the range of from 80 to 85 % by weight.

In another particular embodiment, the topical dressing according to the invention is that where the polyamide has a melting point below 200 °C, in particular, the polyamides have a melting point between 130 °C and below 200 °C. In another particular embodiment, the topical dressing according to the present invention is that where the polyamide comprised in the film is selected from the group consisting of aliphatic homopolyamide, aliphatic copolyamide, partially aromatic homopolyamide, partially aromatic copolyamide, ter-polyamide, and mixtures thereof.

In another particular embodiment, the topical dressing is that where the polyamide is selected from the group consisting of a homopolyamide, and an aliphatic copolyamide obtainable by total or partial polymerization of ε-caprolactam and not crosslinked 2-polyvinyl pyrrolidone (PVP). The term "cross-linking" refers to a bond or a short sequence of bonds that links one polymer chain to another. These links may take the form of covalent bonds or ionic bonds. The term "not cross-linked" when refers to PVP refers to the absence of cross-linking between different polymeric chains of PVP. Not cross-linked PVP has a linear structure and is soluble in water. Thus not-cross linked PVP is equivalent to soluble PVP being the solubility of PVP in water at 25°C $\geq$ 100 mg/ml.

The aliphatic homopolyamide can be selected from the group consisting of polyamide 6 (PA6), polyamide 66 (PA66), polyamide 69, polyamide 610, polyamide 612 (PA612), polyamide 10 (PA10), polyamide 11 (PA11), and polyamide 12 (PA12), polyamide 46, polyamide 1010, and polyamide 1212.

The aliphatic Copolyamide can be selected from the group consisting of: Copolyamide 4/6 (PA4/6), Copolyamide 6/66 (PA6/66), Copolyamide 66/6 (PA66/6),Copolyamide 6/69 (PA6/69); Copolyamide 6/9 (PA6/9), Copolyamide 6/10 (PA6/10), copolyamide 4/10 (PA 4/10), Copolyamide 6/12 (PA6/12); a polyether-amide; a polyester amide; a polyether ester amide; a polyamide urethane; a poly(ether-block-amide). The ter-polyamide can be ter-polyamide 6/66/12.

The partially aromatic polyamide can be selected from the group consisting of polyamide 6-I where I is isophthalic acid, polyamide 6T where T is terephthalic acid, polyamide MXD6 which is a polycondensate of m-xylene diamine and adipic acid (nylon_MXD-6);

The partially aromatic copolyamide can be selected from the group consisting of: Copolyamide 6-I/6-T, Copolyamide 6/6-I, polyamide 4T/6T, polyamide 66/6I and mixtures thereof, wherein I is isophthalic acid and T is terephthalic acid.

In another particular embodiment, the topical dressing according to according to the invention is that where the polyamide is selected from the group consisting of aliphatic copolyamide selected from the group consisting of Copolyamide 4/6 (PA4/6), Copolyamide 6/66 (PA6/66), Copolyamide 66/6 (PA66/6), Copolyamide 6/69 (PA6/69); Copolyamide 6/9 (PA6/9), copolyamide 4/10 (PA 4/10), Copolyamide 6/10 (PA6/10); Copolyamide 6/12 (PA6/12), a polyether amide, a polyester amide, a polyether ester amide, a polyamide urethane, a poly(ether-block-amide), and ter-polyamide 6/66/12 (e.g., Terpalex®). The nomenclature used to name homopolyamides and copolyamides is the one indicated in the norm ISO 1874-1. In another particular embodiment, the topical dressing according to the invention is that where the polyamide is selected from the group consisting of polyamide 6, polyamide 66, copolyamide 6/66, copolyamide 66, copolyamide 6/12, and tert-polyamide 6/66/12.

In a particular embodiment, the topical dressing according to the present invention is that, where the polyvinyl pyrrolidone is in an amount from 8 to 25% by weight. In another particular embodiment, the topical dressing according to the present invention is that where the polyvinyl pyrrolidone is in an amount from 16 to 22% by weight.

In a particular embodiment, the topical dressing according to the present invention, further comprising a plasticizer in an amount equal to or below 14 % by weight. In another particular embodiment, the topical dressing according to the present invention is that where the amount of plasticizer is from 1 to 10% by weight. In another particular embodiment, the topical dressing according to the present invention is that where the plasticizer is in an amount equal to or below 8% by weight. In another particular embodiment, the topical dressing according to the present invention is that where the plasticizer is in an amount equal to or below 5% by weight. In a particular embodiment, the topical dressing according to the present invention is that where the plasticizer is in an amount equal to or below 2% by weight. In another particular embodiment, the topical dressing according to the present invention is that where the amount of plasticizer is from 1 to 2% by weight.

In another particular embodiment, the thermoplastic film according to the invention is that where the plasticizer is selected from the group consisting of: xylitol, glycerin, sorbitol, 1,2-propyleneglycol, pentaerythritol, trimethyl propane, inositol, mannitol, triethanolamine, polyethylene glycol with a molecular weight of 200 to 10,000 atomic mass units (Dalton), mono and diglycol, trimethylolpropane, a mono-, di-, or triester of glycerol with carboxylic acids such as ($C_3$-$C_{12}$)

alkanoic acids, formamide, acetamide, N,N-dimethylformamide, N,N-dimethylacetamide, and mixtures thereof. In another particular embodiment the plasticizer is selected from the group consisting of glycerin, xylitol, and sorbitol. In another particular embodiment, the thermoplastic film is that where the plasticizer is xylitol.

In a particular embodiment, the topical dressing according to the present invention, where the plasticizer is selected from the group consisting of glycerin, xylitol, and sorbitol.

In a particular embodiment, the topical dressing according to the present invention, further comprising an additive selected from the group consisting of a water repellent, a lubricant, a slip agent, an additive for providing color, anti-blocking agent, a mineral filler, a nucleating agent, a UV block additive, a surfactant, and mixtures thereof.

In another particular embodiment, the topical dressing according to the present invention where there is at least an additive which is a water repellent selected from the group consisting of a N,N'-($C_{16}$-$C_{22}$)- alkylenebis amide and triglyceride esters of fatty acids ranging from $C_8$-$C_{30}$; a diglyceride ester of a fatty acid ranging from $C_8$-$C_{30}$, a monoglyceride ester of a fatty acid ranging from $C_8$-$C_{30}$. In a particular embodiment, the N, N'-($C_{16}$-$C_{22}$)-alkylenebis amide is an N,N'-alkylene bisamide of a fatty acid. Examples of N,N'-alkylene bisamides of fatty acids are N,N'-ethylene-bis-stearamide, N,N'-ethylene-bis-oleamide, N,N'ethylene-bis-palmitamide, gadoleamide, erucamide, N,N'-dioleyl adipamide, N,N'-dierucylamide, and stearamides such as ethylene bis stearamide (EBS). EBS can also act as a slip agent, surface lubricant, and antistatic additive. In a particular embodiment, the thermoplastic film is that from those mentioned above or below where the water repellent is EBS. In another particular embodiment, the monoglyceride ester of a fatty acid is the glycerol monostearate.

In another particular embodiment, the topical dressing is that where the anti-blocking agent is selected from the group consisting of calcium carbonate, silica, and talc.

In another particular embodiment, the topical dressing is that where the additive for providing color is selected from the group consisting of inorganic pigments, organic pigments, and colorants.

In another particular embodiment, the topical dressing is that which comprises, a mineral filler which is selected from the group consisting of quartz powder, titanium dioxide, calcium carbonate, talc, mica, aluminosilicates, glass staple fibers, mineral fibers, and microglass beads.

In another particular embodiment, the topical dressing is that where the slip agent is selected from the group consisting of: N,N'-alkylenebisamide, an oleoamide, and a metal salt of stearic acid. Examples of metal salts are those where the metal is calcium, zinc, magnesium, or sodium.

In another particular embodiment, the topical dressing according to the present invention is that where the water repellent is present and is ethylene bis stearamide. In another particular embodiment, the topical dressing according to the present invention is that where the water repellent is in an amount from 0 to 0.5% by weight.

In another particular embodiment, the topical dressing according to the present invention is that where one or more of the layers of the film further comprise an elastomer selected from the group consisting of: polyether block amide copolymer thermoplastic elastomer and polyether ester copolymer thermoplastic elastomer. Both elastomeric copolymers provide flexibility and high-water vapor permeability. In another particular embodiment, the topical dressing as defined above is that where the polyether block amide copolymer thermoplastic elastomer is Pebax® grades by Arkema.

In another particular embodiment, the topical dressing according to the present invention is that where the elastomer is present in an amount from 15 to 40% by weight. In another particular embodiment, the topical dressing according to the present invention is that where the elastomer is in an amount from 15 to 30% by weight. In another particular embodiment, the topical dressing according to the present invention is that where the elastomer is in an amount from 20 to 25% by weight. In another particular embodiment, the topical dressing according to the present invention is that where the elastomer is in an amount of 20% by weight.

In a particular embodiment, the topical dressing as defined above is that further comprising an antimicrobial agent in the surface of the layer to be in contact with the skin.

In another particular embodiment, the topical dressing according to the present invention is that where the antimicrobial agent comprises $Ag^+$. In another particular embodiment the antimicrobial agent is a silver zeolite which is an aluminium porous matrix of sodium silicate treated with Ag (Bactiblock® Masterbatch silver zeolite/PA6 by Laboratorios Argenol SL). This topical dressing is suitable for the prevention and/or treatment of a microbial infection. The antimicrobial agent may also be silver sulfadiazine, silver nitrate, or silver chloride.

In another particular embodiment, the topical dressing according to the present invention is that where the adhesive is an acrylic adhesive or a silicon adhesive. In another particular embodiment the topical dressing according to the present invention is that where the adhesive has a VWTR equal to or higher than the film.

In another particular embodiment, the topical dressing according to the present invention is that where the adhesive is located either on the whole surface of the layer to be in contact with the skin or on the edges of this layer. The topical dressing also comprises a release liner with non-stick properties adhered to the adhesive surface of the film. The liner may be removed, and the topical dressing applied on the skin.

In a particular embodiment, the edges of the topical dressing are of a different material than the film, in particular, when the adhesive is located in the edges.

When the topical dressing comprises an antimicrobial agent, then generally the adhesive is located on the edges of the layer in contact with the skin.

In a particular embodiment, the topical dressing is in the form of rolls. In another particular embodiment, the topical dressing is die cut to desired geometrical shapes and size.

The release liner is typically made of paper, siliconized paper, or plastic that can be coated with a release agent to ensure that the adhesive does not stick to it. When the time comes to use the adhesive product, the user peels off the release liner, exposing the sticky side of the material. In a particular embodiment, the topical dressing according to the present invention is that where the release liner is a siliconized paper. In another particular embodiment, the topical dressing is a roll of self-adhesive topical dressing.

In a particular embodiment, the topical dressing according to the invention is that having a wall thickness ranging from 10 to 150 $\mu$m. In another particular embodiment, the topical dressing according to the invention is that having a wall thickness ranging from 10 to 100 $\mu$m. In another particular embodiment, the topical dressing according to the invention is that having a wall thickness ranging from 15 to 40 $\mu$m. In another particular embodiment, the topical dressing according to the invention is that having a wall thickness ranging from 28 to 35 $\mu$m.

In a particular embodiment the topical dressing of the present invention has one layer. In another particular embodiment the topical dressing is a multilayered structure. In a particular embodiment, the topical dressing of the present invention is that having a multilayered structure which comprises from 2 to 13 layers. In another particular embodiment, the multilayer topical dressing according to the invention may have from 2 to 11 layers. In another particular embodiment, the multilayer topical dressing according to the invention may have from 2 to 7 layers. In another particular embodiment, the multilayer topical dressing has 2 layers. In another particular embodiment, the multilayer topical dressing has 3 layers.

In another particular embodiment, the multilayer topical dressing according to the invention comprises at least two layers, produced by co-extrusion process providing mono or bi-oriented films or non-oriented films when extruded by blown or cast extrusion technology, exhibiting a water vapor transmission rate of at least 170 g/m$^2$/day, preferably, a water vapor transmission rate (WVTR) $\geq$250 g/m$^2$/day, at 23°C and 90/5, in accordance with DIN 53-122. In a particular embodiment, the water vapor transmission rate is in the range from 200 to 1000 g/m$^2$/day. In another particular embodiment, the water vapor transmission rate is in the range from 200 to 500 g/m$^2$/day.

The film of the present invention can be monolithic film. Since it can be monolithic and exhibits low permeability to oxygen, it provides an additional contamination security to the topical dressings.

In a particular embodiment, the topical dressing is that which has a water vapor transmission rate of at least 250 g/m$^2$ per day at 23°C and 90/5 HR% (measured according to a DIN 53-122 using a Permatran equipment from Mocon and measured before sterilization). In another particular embodiment, the packaging is that which has a water vapor transmission rate of at least 400 g/m$^2$ per day.

In another particular embodiment, the topical dressing is that which has an oxygen transmission rate lower or equal to 50 cc/m$^2$ per day.

In another particular embodiment, the topical dressing of the present invention has at least one water vapor permeable layer having a composition according to the present invention for the thermoplastic film of the present invention and at least one high oxygen barrier layer. The at least one high oxygen barrier layer comprises at least a high oxygen barrier polymer. For the purpose of the present invention high oxygen barrier polymer refers to a polymer showing oxygen transmission rate < 10 cc/m2/day/atm measured at 23°C and 50% relative humidity according to DIN 53 380. Example of high oxygen barrier polymers are ethylene vinyl alcohol copolymers (EVOH), Polyvinyl alcohol, and polyvinyl alcohol copolymers.

All the particular or preferred features of the different embodiments disclosed above for the topical dressing are disclosed either alone or in any combination thereof.

It is also part of the present invention the use of a topical dressing as defined above for protecting a wound. This aspect of the present invention may also be formulated as a method of protecting a wound comprising applying the topical dressing according to the present invention to the skin of a human or an animal living body to cover the wound.

When the topical dressing comprises an active pharmaceutical agent, the pharmaceutical active agent is in in contact with the wound. The use of an active pharmaceutical agent for the preparation of a topical dressing comprising it for the treatment of a wound is also part of the present invention.

In a particular embodiment of the topical dressing for use according to the present invention, the topical dressing further comprises an indicator and the treatment further comprises monitoring the indicator of the topical dressing to determine when to replace the dressing.

It is also part of the invention a topical dressing according to the present invention comprising an antimicrobial agent in the surface of the layer to be in contact with the skin, for use in the prevention and/or treatment of a microbial infection. In a particular embodiment, the topical dressing for use according to the present invention is that where the microbial infection is caused by Staphylococcus aureus.

It is also part of the present invention a method of treating a wound comprising applying the wound dressing according to claim 1 to a wound site such that the therapeutic agent is in contact with the wound.

In a particular embodiment, as defined above further comprising monitoring the indicator of the wound dressing to determine when to replace the dressing.

The topical dressings can be prepared by known methods known in the art. The film of the topical dressing of the present invention may be prepared by a process comprising the following steps: a) melt blending the individual components that will form the film which are at least a polyamide in an amount in the range of from 52-92 % by weight, polyvinyl pyrrolidone in an amount in the range of from 8 to 30% by weight; the sum of components of each of the layers being 100% by weight; b) extruding the mixture by a process selected from a mono-orientation process, a bi-orientation process, a blown film not oriented extrusion process, and a cast film not oriented extrusion process; and c) in case of oriented film process, thermally anneal the film, is also considered part of the invention. The film may be mono or bi-oriented or a blown or cast extrusion film which are not oriented.

If the film comprises further components such as a plasticizer in an amount below 14% by weight; an additive selected from the group consisting of a water repellent, a slip agent, an additive for providing color, anti-blocking agent, a mineral filler, a nucleating agent, a UV block additive, a surfactant, and mixtures thereof; or an elastomer; they are melt blended with the polyamide and the PVP.

When the film comprises an antimicrobial agent, it is added, together with the rest of the compounds, to the main feeder to be melt blended.

In a particular embodiment the components are melt blended by means of a twin-screw extruder, at maximum setting extruder temperatures from 210 to 270 °C preferably 220-240 °C. Molten blend mass flows throughout the die extruder holes as filaments which are cooled and cut into pellet shape. In another particular embodiment, the film is produced by the process known as double-bubble process. The film used in the topical dressing of the present invention may be defined by its preparation process, i.e. the film obtainable by any of the processes mentioned above.

It is part of the present invention a film which comprises one or more layers, and each layer comprises a polyamide in an amount from 52-92 % by weight; polyvinyl pyrrolidone in in an amount from 8-30 % by weight, and an elastomer in an amount form 15-40% by weight; and the sum of components in each layer being 100% by weight. All the particular embodiments related to the elastomer in the topical dressing are also particular embodiments for the film.

In a particular embodiment, the topical dressing of the present invention is that which comprises: a) a film; b) a pressure-sensitive biocompatible adhesive on one of the surfaces of the film; and c) a release liner with non-stick properties adhered to the adhesive surface of the film; wherein: the film comprises one or more layers and each layer comprises a polyamide in an amount from 52-92 % by weight; and polyvinyl pyrrolidone in in an amount from 8-30 % by weight; and the sum of components of each of the layers being 100% by weight; and may be prepared by a process comprising the steps of: a) providing the film used in the topical dressing of the invention; b) applying the biocompatible adhesive on one of the surfaces of the film; and c) adding a liner to the adhesive surface of the dressing. In another particular embodiment of the process, the biocompatible adhesive is applied on the edges of one of the surfaces of the film.

In a particular embodiment, the topical dressing of the present invention may be prepared by a process comprising the steps of: a) die-cutting the film used in the topical dressing of the invention into a specific shape and size suitable for the intended application; b) applying the adhesive to the edges of one surface of the die-cut film; and c) adding a liner to the adhesive surface of the dressing;

The adhesive can be applied industrially by different technologies: rotogravure, casting/ doctor bucket, spray, or by a rod.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim and shall not be construed as limiting the scope of the claim. Furthermore, the present invention covers all possible combinations of particular and preferred embodiments described herein.

**Examples**

Example 1: Production of Copolyamide 6/66 - PVP compound

Plasticizer (Xylitol E967, food grade, crystals 10-30mesh, Brenntag) and polyvinyl pyrrolidone (Kollidon K30 by BASF) were fed in the main feeder of a Co-rotating Twin-Screw-Extruder, Leistritz GL27: ∅=27 mm, L/D=36. Co-Polyamide 6/66 UBE NYLON 5024 were dosed by a gravimetric side feeder to the extruder. Total feeding rate was 15kg/h and screws rpm 300. All ingredients were thermally blended and plasticized at melt temperature (actual mass temperature measured with a thermocouple probe in the extruder) of 230-235 °C to obtain through the die filaments which were cooled down previously to be pelletized. Dosing ratio of each ingredient provides 82% Copolyamide 6/66, 16% polyvinyl pyrrolidone and 2% xylitol compound composition.

Example 2: Production of a Bioriented mono layer film-formulation 82% CoPA 6/66 UBE 5024B 16% PVP Kollidon K30; 2% Xylitol

Co-polyamide 6/66 - PVP obtained in Example 1 were fed in a single screw 30mm extruder plasticizing and homogenizing the individual recipe components and forming the melt in an extrusion head into a so-called primary tube. The primary tube was rapidly quenched by means of cold water and pre-heated by hot water, bi-axially oriented, and thermally annealed before winding. This process is well-known as the double-bubble-process with an annealing step or also as triple bubble process. The primary tube was stretched by a factor of 1.9 in machine direction (MD) and by a factor of 2.9 in transversal direction (TD). After being stretched film was heat annealed at 200°C. Final wound tubular film had a wall thickness of 25 µm and 1- 4% thermal shrinkage in its longitudinal and transversal direction. Tubular film was opened to get two flat films by cutting with two knifes both flat tube edges. Film obtained exhibits acceptable transparency and no odor.

Example 3: Production of a monolayer blown film-formulations

Example 3a: Monolayer blown film formulation 82% CoPA 6/66 UBE 5024B 16% PVP Kollidon K30; 2% Xylitol

Same compound obtained in Example 1 was fed together with 1000ppm of EBS (Ethylene bis stearamide Kemfluid 220-G by Union Derivan) in a 20mm single screw extruder where material was thermally plasticized at a melt temperature of 220-225°C. The extrudate melt was fed through a tubular die head of 80mm diameter and 1 mm die gap and immediately inflated up using air inside of formed bubble. Film bubbles were cooled down by chill air and collected and pulled by nip rolls located up and vertically from the die head, cut its lateral edges into two flat films using knifes and finally winding up the film.
30, 50 and 100 µm wall thickness film was obtained with 0-0.5% and 1- 2% thermal shrinkage in its longitudinal and transversal direction with good optical properties and no major issues. Film obtained exhibits acceptable transparency and no odor.

Example 3b Monolayer blown film formulation as Example 3a further additivated with an antimicrobial agent

The film of example 3b is equivalent to the film of example 3a with the addition of a 5% of silver zeolite (Bactiblock® Masterbatch Silver Zeolite/PA6 by Laboratorios Argenol SL). The masterbatch, in form of pellets containing Polyamide 6 as polymer matrix and silver zeolite, is added together with the rest of the compounds, to the main feeder following the same steps of example 1.

Example 4: Compatibility of the film of the present invention with the skin and transpirability

An *in vivo* study was carried out to show the water vapor permeability of the thermoplastic film of the invention. The study was carried out on healthy volunteers and its effect on the skin was evaluated by determining the transepidermal water loss; in order to assess the potential and feasibility of the product for topical and sanitary purposes. The study was carried out under normal conditions of use, under dermatological advice and supervision, by means of instrumental tests on human volunteers. Both for the safety study, for the verification of skin compatibility, and for the quantitative study using dermatological probes, 10 volunteers participated in each of the studies.
Specific inclusion criteria were as follows, for both trials:

- Volunteers: 10
- Age: 18-65 years
- Sex: Both
- Phototype (Fitzpatrick): I to IV (Mixed)
- Skin type: All skin types

The specific exclusion criteria were as follows, for each trial:
Qualitative study or patch test:

- Allergy to nickel
- Allergy or reaction to the same type of products
- Skin hyperreactivity
- Reactivity to adhesive tape

- Severe exposure to the sun at some time during the month prior to the study.
- Intense sun or UVA exposure during the test period
- Sauna baths
- Intensive or regular practice of one or more sports, the practice of which may create difficulties
- Treatment with vitamin A or its derivatives for at least 3 months prior to the start of the study
- Treatment with topical corticosteroids in the experimental area in the 8 days prior to the study.

Quantitative study with dermatological probes:

- Present pathologies in the experimental area.
- Present pathologies that may interfere with the study.
- Present relevant dermatological pathologies (severe acne, atopic dermatitis, psoriasis, lupus, rosacea, ringworm, etc.).
- Volunteers who have undergone organ extraction or transplantation.
- Volunteers with immunodeficiency.
- Volunteers with a history of intolerance to medicines, cosmetics, medical devices, domestic and industrial products.
- Volunteers with a history of allergies, photosensitivity or phototoxicity.
- Volunteers with progressive skin alteration.
- Volunteers with progressive febrile process.
- Volunteers with metabolic photodermatitis: porphyria, tryptophan metabolism disorders.
- Volunteers treated with antibiotics, antihistamines, corticosteroids, beta-blockers, retinoids, azelaic acid, anti-acne treatments or whose treatment has been completed during the 15 days prior to the study.

Methodology: On measurement days, the volunteers who participated in the studies remained for 10-15 minutes in an acclimatized room before each of the measurements were taken.

Qualitative study or patch test:

To verify the skin compatibility of the film in example 3b after a single application on the skin and under an occlusive patch for 48 hours and under follow-up by the dermatologist.
Type of study: Skin compatibility study under occlusive patch.
Test conditions: Both in the waiting room and in the dermatologist's office, the conditions were as follows:

Table 1. Climatic conditions in dermatologist's rooms.

| Location | Temperature | Humidity |
|---|---|---|
| Waiting Room | 25 ± 4.0 °C | 46 ± 2 % |
| Doctor's Office | 24.0 ± 4.0 °C | 45 ± 2 % |
| - Duration of the study: 48 h (plus approx. 30 minutes of reading, after removing the patch).<br>- Area of application: back.<br>- Patch type: Finn Chambers Aqua<br>- Application conditions: Product applied on the aluminum dome.<br>- Quantity: 50 mm$^2$ | | |

Evaluation:

- Parameters analyzed: erythema, edema, papules, vesicles, blisters, necrosis, pruritus.
- Irritation Index Grading:

  ◦ Negative, doubtful, weak, strong, and extreme.
  ◦ Suggested values:

  ▪ Negative: 0
  ▪ Doubtful: 1
  ▪ Weak Erythema: 2

# EP 4 640 246 A1

- Strong: 3
- Extreme: 4

- Evaluation: The average of all the results obtained was averaged and evaluated as follows:

Table 2. Interpretation of Results According to the Reaction Index (RI).

| Results interpretation | |
|---|---|
| 0≤RI≤1 | Tolerated |
| 1≤RI≤2 | Slightly Tolerated |
| 2≤RI≤3 | Irritant |
| 3≤RI≤4 | Very Irritant |

Table 3. Results of the qualitative study or patch test

| Volunteer | Classification of irritation | | | | |
|---|---|---|---|---|---|
| | Negative | Doubtful | Weak | Strong | Extreme |
| 1 | X | | | | |
| 2 | X | | | | |
| 3 | X | | | | |
| 4 | X | | | | |
| 5 | X | | | | |
| 6 | X | | | | |
| 7 | X | | | | |
| 8 | X | | | | |
| 9 | X | | | | |
| 10 | X | | | | |
| Score | 10 | 0 | 0 | 0 | 0 |
| Reaction rate value | 0.0 | 0.0 | 0.0 | 0.0 | 0.0 |

Table 4. Interpretation of the results of the qualitative study or patch test

| Interpretation of results | |
|---|---|
| 0≤Reaction rate≤1 | Tolerated |

From the results obtained under the experimental conditions adopted, it can be concluded that the film of example 3b has very good skin compatibility.

Quantitative Study with Dermatological Probes:

In the present study, the film of example 3b and a commercial film C (Tegaderm roll of 3M; Ref: 16004S) were used. Film C is a Control film, not of the invention.

Test conditions:

In both the waiting room and the dermatologist's consultation room, the conditions were the following:

Table 5. Climatic Conditions in Dermatologist's Rooms

| Location | Temperature | Humidity |
|---|---|---|
| Waiting Room | 22,2 ± 4,0 °C | 43 ± 2 % |
| Doctor's Office | 22,0 ± 4,0 °C | 42 ± 2 % |

The duration of the study was 2 hours with an extra of 30 minutes of reading the results, after removing the patches. The area of application was facial, specifically the forehead. Two films were positioned across the forehead: one on the left side and one on the center. The right side was reserved for control, all aligned horizontally. The film of example 3b was placed on the left area of the subject's forehead and fixed with adhesive tape on the edges. In the central area, the commercial film was placed in the same way. For the control area, the right zone was demarcated, matching the size of the films and left uncovered, to serve as a control.

The films were placed without folds or wrinkles and fixed with adhesive tape to avoid leaks or possible loss of water vapor from the skin. Contact with capillary areas was also avoided.

Experimental procedure and instrumentation:

For the in vivo studies, a MPA multi-probe system equipment was used, consisting of 8 probes, to analyze the skin and control of environmental conditions. Specifically for this study, the following probes were used: TEWAMETER TM300 probe. With this probe, the transepidermal water loss (TEWL) in $(g/h/m^2)$ was determined.

This probe quantifies the deep hydration of the skin by assessing the transepidermal water loss from it, before and after using the films. In addition, the probe was used to determine the water vapor flux through the films once placed on the skin.

Measurements were initially taken at time zero (t0) on the right, left, and middle areas of the forehead, designated for the placement of the control, example 3b film and the commercial film, respectively, with clean and dry skin. Subsequently, measurements were taken on the left and middle areas immediately after placing the films (t0f!ash), after 1 hour (t1), and after 2 hours (t2). Finally, after removing the films, measurements were taken again (t2final) on the uncovered skin, in the left, right, and middle areas.

The skin PH-METER 905 probe was used as a measuring instrument to determine the skin's pH.

Results are obtained in pH values (scale 0-14) at time zero on uncovered skin, in the three areas, and at 2 hours, after removing the films in the same areas.

Results of the Quantitative Study with Dermatological Probes

Before analyzing the results of the TEWAMETER probe, it should be considered that once the film has been removed, a lower water vapor flow rate can be expected if the film has transpired heavily; while if the film transpires less, moisture can accumulate between the skin and the film. This results in a higher water vapor flow rate once the film has been removed.

The average data obtained with the TEWAMETER probe and the comparative results of film of example 3b and film C at different times are shown below.

Table 6. Mean TEWL data on the skin of the subjects (before placing the films and after removal) in the areas of the films, both Example 3b and film C at time 0 (t0) and after 2 hours (t2final).

| TEWL $(g/h/m^2)$ | | | |
|---|---|---|---|
| Example 3b | | Film C | |
| $t_0$ | $t_2$final | $t_0$ | $t_2$final |
| 15,39 | 24,82 | 14,45 | 30,66 |

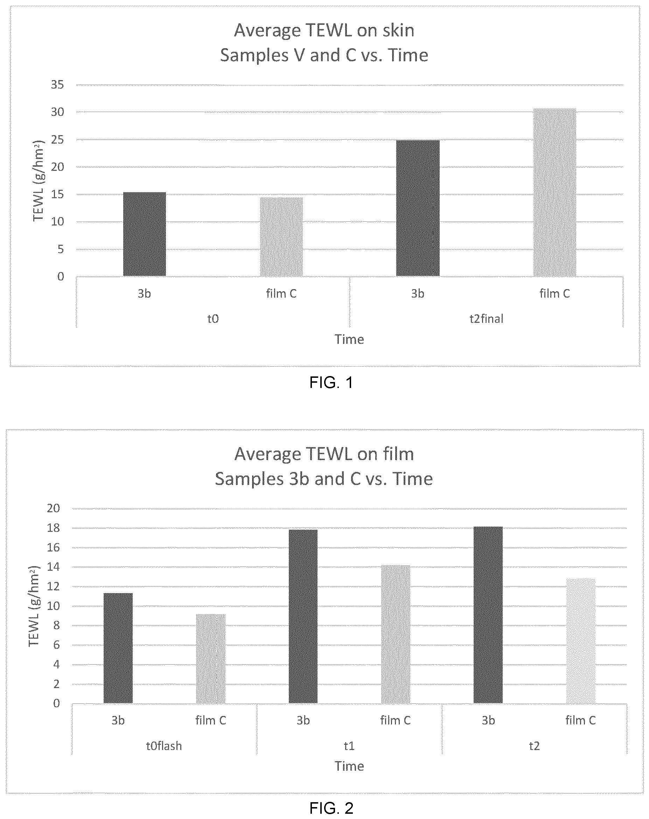

According to the averages of the TEWL data obtained (see as well in FIG. 1), the transepidermal water vapor loss, once the films have been removed, is greater in the area where the Commercial film was installed, compared to the loss obtained in the area where the example 3b was placed.

Table 7. Mean data of TEWL measurements taken on films placed on the skin of subjects in the 3b and Commercial film C areas at times t0f!ash (immediately after placing the films), 1 and 2 hours.

| TEWL (g/h/m$^2$) | | | | | |
|---|---|---|---|---|---|
| Example 3b | | | Film C | | |
| $t_0$flash | $t_1$ | $t_2$ | $t_0$flash | $t_1$ | $t_2$ |
| 11,13 | 17,81 | 18,13 | 9,17 | 14,19 | 12,84 |

As for the data obtained with the same probe, but obtained with the measurements on the films placed on the skin of the subjects at different times, it is observed that, in all cases, the film 3b has a higher water vapor flow rate, which means that the water vapor permeability of the 3b film is greater than that of the commercial film C (see as well in FIG. 2).

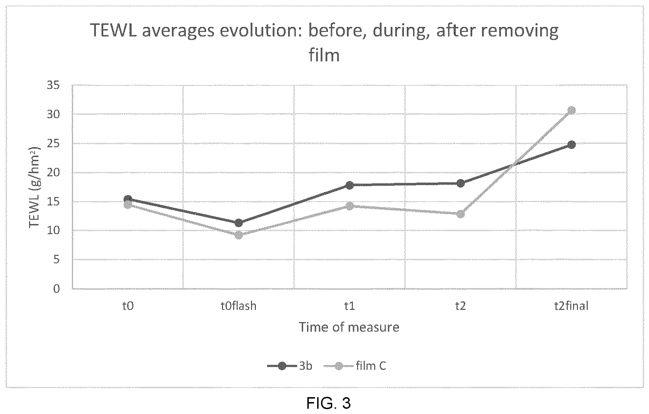

From FIG. 3 it can be seen the differences between the means of the TEWL measures of the example 3b and film C.

The TEWL values of the averages obtained at t0f!ash (when placing the films on the skin) with respect to t0, have decreased, due to the fact that the initial measurements are made on the skin and those at t0f!ash on the films. This is due to the shielding of the films themselves. And, as can be seen at t0f!ash, from the beginning there are differences between the example 3b and the commercial film C; Example 3b has a higher water vapor flow.

On the other hand, both after 1 hour and 2 hours of application of the films, the example 3b film shows greater water vapor permeability in the form of water vapor flow or TEWL, compared to the commercial film C.

Finally, after 2 hours of application and having removed both films, there is a considerable increase in TEWL averages. This phenomenon is due to the release of excess moisture retained by the skin after having been covered by a film for a certain period of time. In addition, at this same point, it can be observed that example 3b had produced less moisture than the commercial film C.

A statistical analysis of the results has been carried out over the data obtained in the quantitative study with subjects, using the dermatological probe Tewameter with which measurements were made on the skin before placing the films, on the films themselves during the application and once they were removed, again on the skin.

For this purpose, statistical methods have been applied to related groups in order to obtain an objective evaluation of the information contained in the data. The selection of the statistical method depends on the number of study variables or factors, as well as other parameters such as normality, absence of outliers, sphericity, and others. The software used to perform this analysis has been JAMOVI.

The statistical analysis has been divided into 2 parts: On one hand, the measurements made on the user's skin at time 0 before placing the films (t0) and after removing them 2 hours later (t2final); to evaluate how this has affected the subjects, the application of the films after 2 hours of use. On the other hand, the transepidermal water loss or TEWL has been evaluated, measuring on the films recently placed on the skin (t0f!ash time) and after 1 and 2 hours.

After the experimental observations and the statistical analysis carried out on the TEWL data, it can be affirmed that, with regard to the TEWL measurements obtained on the user's skin:
There are no differences at time t0 between the two samples evaluated films 3band C (p=0.432 with the non-parametrical Wilcoxon statistical method). There are significant differences for sample 3b between time $t_0$ and time t2final (TEWL worsens). There are significant differences for sample C between t0 and t2final times (TEWL worsens).

There are statistically significant differences at time t2final between samples 3b and C, with the TEWL value being higher for sample C. This last conclusion may indicate that, due to the lower transpiration of C, the TEWL is retained. This latter conclusion may indicate that, due to the lower transpiration of C, more moisture is retained between the film and the skin and, after removing the film, there is a much higher evaporation of moisture in sample C.

As for the TEWL measurements, obtained on the films: At time t0f!ash there are no significant differences between the TEWL of Example 3b and Film C (p=0.232 with the non-parametrical Wilcoxon statistical method). At time t1 there are significant differences between the TEWL of Example 3b and Film C; in this case the TEWL of Example 3b is higher (as the transpiration of the 3b film is higher than that of the commercial film C) (p=0.01 with the non-parametrical Wilcoxon statistical method).

At t2 time, there are significant differences between the TEWL of Example 3b and Film C; Example 3b being also higher (as the transpiration of 3b film is still higher than the commercial film C) (p=0.028 with the non-parametrical Wilcoxon statistical method). As for the averages of the results obtained with the pH-METER probe, in the areas where the films (3b and Commercial film C) were placed before being placed and after being removed 2 hours later, the following data was collected:

Table 8. Mean data of the results obtained with the pH-Meter on the skin of the subjects, in the different areas of the test, at the beginning and end of the test.

| | pH (1-14) | |
|---|---|---|
| | $t_0$ | $t_2$ |
| Left area | 6.27 | 5.54 |
| Middle area | 6.17 | 5.56 |
| Right area | 6.38 | 5.45 |

Based on the skin reactions observed, it can be stated that none of the volunteers included in the study presented skin pathology of an inflammatory or infectious nature around the area of application of the product at the first visit and were therefore included in the study. In conclusion, the overall skin safety profile of the tested product has not been compromised and the product has good skin compatibility.

After the results obtained with the probes and the statistical analysis of the data obtained with the Tewameter probe to determine the transpirability of the skin and the water vapor permeability of the example 3b compared to a commercial film, whose behavior is also shown in the previous figures, the following conclusions have been reached: Instantly (t0f!ash), when both example 3b and the commercial film C are applied, the water vapor flow rate decreases comparing the values obtained on both films with respect to the uncovered skin at time 0. After 1 hour of the films being placed on the skin, statistically significant differences are already found between films 3b and C; presenting higher water vapor flow values in the example 3b, compared to the Commercial film.

After 2 hours of use, both films suffer a slight decrease in the water vapor flow rate possibly due to the stabilization of the water vapor permeability of the skin; although a statistically significant difference is still obtained between the two films, the film from example 3b continues to provide greater water vapor permeability. After 2 hours and once the films have been removed, there is an increase in the values recorded for transepidermal water loss on the skin, with a notable increase in the case of the Commercial film C. This is due to the shielding effect produced by the films themselves on the skin, creating an excess of moisture between the films and the skin. When the film is removed, there is a high evaporation of moisture from the skin. It is noteworthy that, in the case of the example 3b, this shielding effect was much less, thus corroborating its greater water vapor permeability compared to the commercial film. This difference has also been analyzed statistically, and a significant difference has been obtained.

Example 5: Antibacterial activity

A culture of Staphylococcus aureus strain CECT 240 was prepared, with a concentration between $2.5\text{-}10 \times 10^5$ cfu/ml. The concentration was verified by microbiological analysis by plate count. Each of the test specimens was placed on a petri dish, on which an inoculum of the culture is placed. The inoculum of the prepared bacterial culture was placed on top of each test specimen. Each test specimen is covered with a 40x40 mm plastic film covering part of the surface of each test tube together with the inoculum, the inoculum of bacteria and incubate the Petri dishes with the test tubes. The Petri dishes with the specimens inside were incubated in a climatic chamber at 37°C, 100% humidity, for 24 hours, passing through the incubation chamber after which the deposited bacteria were extracted as follows: Extraction: Each of the test tubes together with its corresponding film was placed in a sterile bag with 50 ml of sterile water and then it was placed in the stomacher for 1 minute.

Each of the extracts generated from the extraction of all samples is collected in sterile tubes and a plate count of the bacteria in each sample is carried out.

The results were obtained as colony forming units (cfu) and log (log), per $cm^2$ of contact surface tested. The evaluation of the results was carried out through the calculation of the antibacterial activity according to the following formula:

$$R = (Ut - U0) - (At - U0) = Ut - At$$

where:

R = Value of antibacterial activity
Ut: Mean, in logarithm, of viable bacteria recovered per test specimen area tested ($cm^2$), in the test specimens ($cm^2$), in the control specimens at 24 hours (or viability at 24 hours). In this case it is compared against the viability of S. aureus bacteria at 24 hours.
U0: Mean in logarithm of viable bacteria recovered per control test tube surface (cm2), immediately after inoculation.

At= Mean, in logarithm, of viable bacteria recovered per test tube area (cm$^2$), on test tube treated for 24 hours.

The results obtained, against Staphylococcus aureus, are shown in the following table:

Table 9: Biocidal activity against Staphylococcus aureus

| References | t = 0 h | | t = 24 h | | R |
|---|---|---|---|---|---|
| | ufc/cm$^2$ | log/cm$^2$ | ufc/cm$^2$ | log/cm$^2$ | |
| S. aureus viability | 1.83E+04 | 4.3 | 6.7E+04 | 4.8 | |
| Example 3a | | | 8.23E+04 | 4.9 | 0 |
| Example 3b | | | 1.51E+01 | 1.2 | 3.6 |

Concentration of inoculated Staphylococcus aureus: $7.3 \times 10^5$ cfu/ml.

It is considered that a surface has antibacterial activity if the R value is greater than or equal to 2, compared to the control (bacterial viability in this case). Therefore, under the test conditions described in the report, and according to the obtained results: The reference samples of example 3b present antibacterial activity against Staphylococcus aureus. Example 3a does not show antibacterial activity against Staphylococcus aureus.

Example 6: Evaluation of the properties of the films used in the topical dressings of the present invention

Materials:

Pebax® MH 1657: Polyether block amide thermoplastic elastomer by Arkema
MSG: Glycerol monostearate- Einar 204 supplied by Palsgaard
Xylitol: Food grade xylitol VF0234 supplied by Vitalfoods
PVP: Water soluble PVP- Kollidon K30 by Basf
CoPA: UBE Nylon 5024 by UBE Copolyamide 6/66
EBS (Ethylene bis stearamide Kemfluid 220-G by Union Derivan)

The films have been prepared following the procedure disclosed in Example 3. Measuring methods used for determining the following parameters of the films used in the topical dressings of the present invention.

Water vapor transmission rate was determined in accordance with DIN 53-122 at 23°C 90/5 %relative humidity using a Permatran-W® permeation analyzer by Mocon. 90/5 % relative humidity is the % humidity in the nitrogen stream flowing continuously above/below respectively the sample film to be evaluated in the water vapor permeation equipment which for the present invention is Permatran from Mocon. It can also be expressed as 85% relative humidity which is the difference or gradient between the two streams.

Oxygen transmission rate was analyzed in accordance with DIN 53 380 at 23°C and 50% relative humidity using an Ox-Tran® permeation analyzer by Mocon.

MD and TD tensile strength and MD and TD tensile elongation were measured in accordance with ISO 527-3 using a universal testing equipment by Instron 3365.

Maximum puncture force was assessed in accordance with internal test method using a universal testing equipment Instron 3365.

Conditioning of sample at 23°C@50% RH for 8 hours (at least).
Size of sample: diameter 80mm
Cell load: 1000N
Speed: 500 mm/min
Blunt tip. flat tip with diameter of 2,5mm.

Sample with diameter of 80 mm is mounted in sample holder. Tip is moved down in order to gently touch the surface of measured film with initial force ≤ 0,5N. Measurement is started under conditions described above.

Max force (N) for puncture is recorded. At least 6 measurements per sample are carried out.

MD and TD tearing force was assessed by internal method using a universal testing equipment Instron 3365

Size of sample: length 80mm
Cell load: 100N
Speed: 100 mm/min
Grip distance: 40mm

Sample with length of 80mm and width of 30mm is partially cut on half (length of partial cut = 40mm, width of cut= 15mm) and it is mounted between clamps (initial cut is parallel to direction of elongation) where grip distance is 40mm. Initial force (elongation=0%) should be between 0,1N and 0,5N.

Conditioning of sample at 23°C@50% RH for 8 hours (at least).

Measurement is started under conditions described above.

Maximal force (N) for tearing is recorded. At least 6 measurements per sample are carried out. Measurements are done at both directions (MD and TD).

Wall thickness of the thermoplastic film is measured with a micrometer.

Blown film properties

| Variable analyzed | # sample | CoPA UBE 5024 % | PVP % | Xylitol % | PEBAX MH 1657 % | EBS/MSG % | WVTR g/m²/d |
|---|---|---|---|---|---|---|---|
| | Example 2 | 82 | 16 | 2 | 0 | 0 | 356 |
| - | Example 3 | 82 | 16 | 2 | 0 | 0.1/0 | 300 |
| PVP concentration | Example 6a | 76 | 22 | 2 | 0 | 0 | 421 |
| | Example 6b | 90 | 8 | 2 | 0 | 0 | 176 |
| Xylitol concentration | Example 6c | 84 | 16 | 0 | 0 | 0 | 310 |
| | Example 6d | 74 | 16 | 10 | 0 | 0 | 431 |
| MSG | Example 6e | 72 | 16 | 10 | 0 | 0/2 | 265 |
| PEBAX MH 1657 | Example 6f | 65,5 | 13 | 1,5 | 20 | 0 | 390 |

| Variable analyzed | # sample | Film thickness (μm) | OTR (cm³/m²/day) | Tensile strength MD/TD (MPa) | Tensile elongation MD/TD (%) | Film Puncture Force max. (N) | Film Tearing Force MD/TD (N) |
|---|---|---|---|---|---|---|---|
| - | Example 2 | 25 | 29 | 152/159 | 137/82 | 29 | 20/10 |
| - | Example 3 | 31 | 31 | 71/63 | 421/470 | 15 | 17/17 |
| PVP conc. | Example 6a | 34 | 31 | 53/58 | 367/373 | 17 | 17/17 |
| | Example 6b | 30 (25-34) | 31 | 54/49 | 396/381 | 13 | 17/17 |
| Xylitol conc. | Example 6c | 35(30-40) | 33 | 51/51 | 385/382 | 14 | 16/17 |
| | Example 6d | 31(26-37) | 33 | 49/41 | 420/375 | 11 | 18/18 |
| MSG | Example 6e | 29 (25-33) | 38 | 55/46 | 463/432 | 14 | 17/18 |
| PEBAX MH 1657 | Example 6f | 31 | 50 | 62/43 | 434/353 | 14 | 17/17 |
| * conc. refers to concentration expressed as a weight percentage of the total weight of the composition. | | | | | | | |

**Citation List**

Patent Literature

- WO2011022680A2
- EP1380212B1
- WO20231803231A1.

**Claims**

1. A topical dressing comprising a film, wherein the film comprises one or more layers and each layer comprises a polyamide in an amount from 52-92 % by weight; and polyvinyl pyrrolidone in in an amount from 8-30 % by weight; and the sum of components in each layer being 100% by weight.

2. The topical dressing according to claim 1, further comprising:

   a) a pressure-sensitive biocompatible adhesive on one of the surfaces of the film; and
   b) a release liner with non-stick properties adhered to the adhesive surface of the film.

3. The topical dressing according to any of the claims 1-2, wherein the film comprises multiple layers and the adhesive is on the surface of the layer to be in contact with the skin.

4. The topical dressing according to any of the claims 1-3, wherein the polyamide is in an amount from 70 to 90% by weight.

5. The topical dressing according to any of the claims 1-4, wherein the polyvinyl pyrrolidone is in an amount from 8 to 25% by weight.

6. The topical dressing according to any of the claims 1-5, wherein one or more of the layers of the film further comprise a plasticizer in an amount equal to or below 14% by weight.

7. The topical dressing according to any of the claims 1-6, wherein one or more of the layers of the film further comprise an additive selected from the group consisting of: a water repellent, a lubricant, a slip agent, an additive for providing color, anti-blocking agent, a mineral filler, a nucleating agent, a UV block additive, a surfactant, and mixtures thereof.

8. The topical dressing according to claim 7, wherein the water repellent is present and is selected from the group consisting of a N,N'-$(C_{16}$-$C_{22})$-alkylenebis amide, a triglyceride ester of a fatty acid ranging from $C_8$-$C_{30}$; a diglyceride ester of a fatty acid ranging from $C_8$-$C_{30}$, a monoglyceride ester of a fatty acid ranging from $C_8$-$C_{30}$

9. The topical dressing according to any of the claims 1-8, wherein one or more of the layers of the film further comprise an elastomer selected from the group consisting of:
   polyether block amide copolymer thermoplastic elastomer and polyether ester copolymer thermoplastic elastomer.

10. The topical dressing according to claim 9, wherein the elastomer is present in an amount from 15-40% by weight in the film.

11. The topical dressing according to any of the claims 1-10, further comprising an antimicrobial agent in the surface of the layer to be in contact with the skin.

12. The topical dressing according to claim 11, wherein the antimicrobial agent comprises Ag+.

13. Use of a topical dressing as defined in any of the claims 1-12, for protecting a wound.

14. A topical dressing as defined in any of the claims 11-12, for use in the prevention and/or treatment of a microbial infection.

15. A film which comprises one or more layers, and each layer comprises a polyamide in an amount from 52-92 % by weight; polyvinyl pyrrolidone in in an amount from 8-30 % by weight, and an elastomer in an amount form 15-40% by weight; and the sum of components in each layer being 100% by weight.

FIG. 1

FIG. 2

FIG. 3

Europäisches
Patentamt

European
Patent Office

Office européen
des brevets

# EUROPEAN SEARCH REPORT

Application Number

EP 24 38 2462

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | WO 2023/180323 A1 (VISCOFAN ESPANA S L U [ES]) 28 September 2023 (2023-09-28)<br>* claim 1 *<br>----- | 15<br><br>1-14 | INV.<br>A61L15/22<br>A61L15/44<br>A61L15/46 |
| A | GB 906 911 A (ROLAND WAGNER) 26 September 1962 (1962-09-26) * page 2, left-hand column, lines 23-26, 43-50 *<br>----- | 1-15 | |
| A | US 2013/324922 A1 (QUINT BODO [CH] ET AL) 5 December 2013 (2013-12-05) * claims 1, 3 *<br>----- | 1-15 | |

**TECHNICAL FIELDS SEARCHED (IPC)**

A61L

The present search report has been drawn up for all claims

| Place of search | Date of completion of the search | Examiner |
|---|---|---|
| The Hague | 3 October 2024 | Dudás, Eszter |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 24 38 2462

This annex lists the patent family members relating to the patent documents cited in the above-mentioned European search report.
The members are as contained in the European Patent Office EDP file on
The European Patent Office is in no way liable for these particulars which are merely given for the purpose of information.

03-10-2024

| Patent document cited in search report | | Publication date | Patent family member(s) | | Publication date |
|---|---|---|---|---|---|
| WO 2023180323 | A1 | 28-09-2023 | NONE | | |
| GB 906911 | A | 26-09-1962 | BE | 606280 A | 16-11-1961 |
| | | | DE | 1190608 B | 08-04-1965 |
| | | | GB | 906911 A | 26-09-1962 |
| US 2013324922 | A1 | 05-12-2013 | CA | 2816573 A1 | 28-06-2012 |
| | | | CN | 103249435 A | 14-08-2013 |
| | | | EP | 2654818 A1 | 30-10-2013 |
| | | | ES | 2674427 T3 | 29-06-2018 |
| | | | JP | 5848776 B2 | 27-01-2016 |
| | | | JP | 2014501587 A | 23-01-2014 |
| | | | US | 2013324922 A1 | 05-12-2013 |
| | | | WO | 2012084390 A1 | 28-06-2012 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011022680 A2
- EP 1380212 B1

- WO 20231803231 A1